(19) Europäisches Patentamt
European Patent Office
Office européen des brevets

(11) Veröffentlichungsnummer: **0 024 673**
**B1**

(12) # EUROPÄISCHE PATENTSCHRIFT

(45) Veröffentlichungstag der Patentschrift: **20.07.83**

(51) Int. Cl.³: **C 07 D 233/50**, A 61 K 31/415

(21) Anmeldenummer: **80104917.2**

(22) Anmeldetag: **19.08.80**

(54) **2-(2-Chlor-4-cyclopropyl-phenyl-imino)-imidazolidin, dessen Säureadditionssalze, diese enthaltende Arzneimittel und Verfahren zur Herstellung derselben.**

(30) Priorität: **22.08.79 DE 2933930**

(43) Veröffentlichungstag der Anmeldung: **11.03.81 Patentblatt 81/10**

(45) Bekanntmachung des Hinweises auf die Patenterteilung: **20.07.83 Patentblatt 83/29**

(84) Benannte Vertragsstaaten: **BE IT**

(56) Entgegenhaltungen:

**EP-A-0 007 422**
**EP-A-0 012 822**

**JOURNAL OF MEDICINAL CHEMISTRY. Band 19, Nr. 8, 1976 B. ROUOT et al. «Clonidine and Related Analogues. Quantitative Correlations» Seiten 1049 bis 1054**

**ARZNEIM.-FORSCH. (DRUG RES.), Band 25, Nr. 5, 1975 W. HOEFKE et al. «Relationship between Activity and Structure in Derivatives of Clonidine» Seiten 786 bis 793**

**ARZNEIM.-FORSCH. (DRUG RES.), Band 25, Nr. 10, 1975 D. COSNIER et al. «Pharmacological Properties of 2-(2-Chloro-p-toluidino)-2-imidazoline-nitrate (Tolonidine), a New Antihypertensive Agent» Seiten 1557 bis 1561**

(73) Patentinhaber: **C.H. BOEHRINGER SOHN, Postfach 200, D-6507 Ingelheim am Rhein (DE)**

(72) Erfinder: **Stähle, Helmut, Dr., Rotweinstrasse 23, D-6507 Ingelheim/Rhein (DE)**
Erfinder: **Köppe, Herbert, Dr., Neuweg 72, D-6507 Ingelheim/Rhein (DE)**
Erfinder: **Kummer, Werner, Dr., Georg-Scheuing-Strasse 15, D-6507 Ingelheim/Rhein (DE)**
Erfinder: **Hoefke, Wolfgang, Dr., Rosselstrasse 21, D-6200 Wiesbaden (DE)**
Erfinder: **Pichler, Ludwig, Dr., Gusshausstrasse 24, A-1040 Wien (AT)**

**EP 0 024 673 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## 2-(2-Chlor-4-cyclopropyl-phenyl-imino)-imidazolidin, dessen Säureadditionssalze, diese enthaltende Arzneimittel und Verfahren zur Herstellung derselben

Aus J. Med. Chem. *19* (1976) Seiten 1049 bis 1054 sind eine Reihe von Phenylimino-imidazolidinen bekannt, die in ihrem Phenylteil durch verschiedenartige Substituenten, wie z.B. Halogen-, Alkoxy- oder Alkylreste strukturell abgewandelt sind. Unter diesen Vertretern erlangten das 2,6-Dichlorphenylimino-imidazolidin, das 2-Chlor-4-methylphenyl- bzw. 4-Methylphenylimino-imidazolidin eine grössere Bedeutung zur Senkung des Blutdruckes und/oder der Herzfrequenz.

Arzneistoffe, die einen Cyclopropylphenylrest in ihrer chemischen Struktur enthalten, sind gleichfalls bekannt, z.B. Procinolol in M. Negwer: Org.-Chem. Arzneimittel, Berlin (1978), Seite 518, Nr. 2889.

Die vorliegende Erfindung betrifft nun 2-(2-Chlor-4-cyclopropyl-phenyl-imino)-imidazolidin der Formel

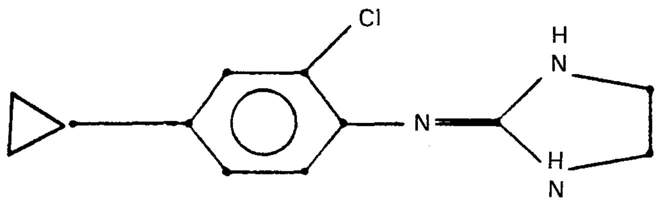

sowie dessen physiologisch verträgliche Säureadditionssalze mit überraschenden wertvollen therapeutischen Eigenschaften.

Aufgrund pharmakologischer Testergebnisse konnte nachgewiesen werden, dass die erfindungsgemässen Verbindungen ein unerwartet besseres Wirkungsprofil im Vergleich zu den nächststehenden bekannten Imidazolidine-Derivaten aufweist.

Die Herstellung der Verbindungen der Formel I erfolgt durch

a) Umsetzung einer Verbindung der allgemeinen Formel

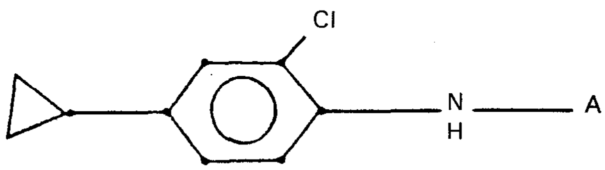

II

in der A eine Cyanogruppe oder den Rest 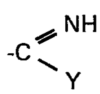 bedeutet, wobei Y eine Alkoxy- oder Alkylthiogruppe mit bis zu 4 C-Atomen oder eine Sulfhydryl- oder Aminogruppe darstellt, mit Äthylendiamin oder dessen Säureadditionssalzen.

Die Umsetzung erfolgt bei Temperaturen zwischen 100 und 250°C. Als Lösungsmittel können polare protische, polare aprotische oder unpolare verwendet werden. Die Umsetzung kann aber auch ohne Anwendung von Lösungsmitteln bei erhöhter Temperatur erfolgen. Die Reaktionszeit schwankt zwischen einigen Minuten und mehreren Stunden.

b) Umsetzung einer Verbindung der Formel

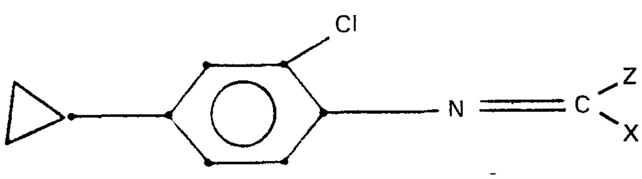

III

worin X und Z, die gleich oder verschieden sein können, ein Halogenatom oder eine Alkoxy- oder Alkylthiogruppe mit bis zu 4 C-Atomen bedeuten, mit Äthylendiamin.

Wenn X und Z ein Halogenatom, bevorzugt ein Chloratom, bedeuten, erfolgt die Umsetzung bei Temperaturen zwischen 0°C und Raumtemperatur. Als Lösungsmittel können inerte Solventien wie Äther, Ketone, Ester oder aliphatische oder aromatische Kohlenwasserstoffe verwendet werden.

Wenn X und Z Alkoxy- oder Alkylthiogruppen bedeuten, verläuft die Umsetzung bei erhöhter Temperatur, vorzugsweise Rückflusstemperatur. Als Lösungsmittel können polare protische, polare aprotische oder unpolare verwendet werden.

c) Abspaltung des Acylrestes aus einer Verbindung der Formel

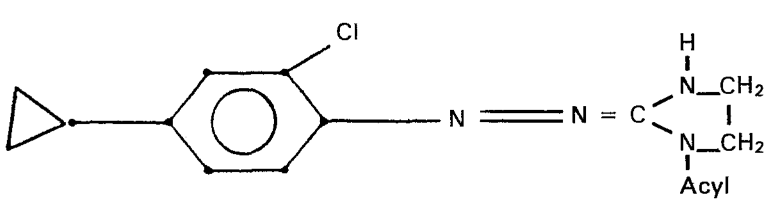

IV

worin Acyl eine aliphatische oder aromatische Acylgruppe bedeutet, mittels aliphatischer Alkohole oder verdünnter Säuren.

Verbindungen der Formel II erhält man ausgehend von 2-Chlor-4-cyclopropylanilin durch Umsetzung mit Cyanaten oder Thiocyanaten, wobei Harnstoffe bzw. Thioharnstoffe gebildet werden. Harnstoffe und Thioharnstoffe können dann weiter mit Alkylierungsmitteln in entsprechende Isouroniumsalze bzw. Isothiouroniumsalze überführt werden. Aus diesen Säureadditionsverbindungen lassen sich mit Basen der entsprechende Isoharnstoff bzw. Isothioharnstoff gewinnen. Durch Wasserabspaltung aus dem Harnstoff bzw. $H_2S$-Abspaltung aus dem Thioharnstoff mittels Blei- oder Quecksilbersalzen gelangt man zum 2-Chlor-4-cyclopropylphenylcyanamid, an das Ammoniak unter Bildung von 2-Chlor-4-cyclopropylphenylguanidin angelagert werden kann.

Das Isocyaniddichlorid der Formel III ist durch Umsetzung von 2-Chlor-4-cyclopropyl-anilin mit Ameisensäure und weitere Reaktion des gebildeten Formanilids mit einem Gemisch aus Thionylchlorid und Sulfurylchlorid zugänglich.

Durch Umsetzung des Isocyaniddichlorids mit Alkoholen oder Thioalkoholen erhält man weitere Ausgangsverbindungen der Formel III.

Ausgangsverbindungen der Formel IV werden durch Umsetzung von 2-Chlor-4-cyclopropyl-anilin mit N-Acyl-imidazolidinonen-(2) in Gegenwart von Phosphoroxychlorid hergestellt.

Das erfindungsgemässe 2-Phenylimino-imidazolidin der Formel I kann auf übliche Weise in seine physiologisch verträglichen Säureadditionssalze überführt werden. Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Salpetersäure, Essigsäure, Propionsäure, Buttersäure, Capronsäure, Valeriansäure, Oxalsäure, Malonsäure, Bernsteinsäure, Maleinsäure, Fumarsäure, Milchsäure, Weinsäure, Zitronensäure, Äpfelsäure, Benzoesäure, p-Hydroxybenzoesäure, p-Aminobenzoesäure, Phthalsäure, Zimtsäure, Salicylsäure, Ascorbinsäure, Methansulfonsäure, 8-Chlortheophyllin und dergleichen.

Die neue Verbindung der Formel I und ihre Säureadditionssalze wirken sehr stark bradycard und sind daher zur Therapie von Coronarerkrankungen geeignet. Die Beeinflussung der Herzfrequenz wurde an Kaninchen und an Spinalratten sowie an intakten, narkotisierten Ratten untersucht.

Die Dosierung liegt bei 0,1 bis 80 mg, vorzugsweise 1 bis 30 mg.

Die Verbindung der Formel I bzw. ihre Säureadditionssalze können auch mit andersartigen Wirkstoffen zum Einsatz gelangen. Geeignete galenische Darreichungsformen sind beispielsweise Tabletten, Kapseln, Zäpfchen, Lösungen oder Pulver; hierbei können zu deren Herstellung die üblicherweise verwendeten galenischen Hilfs-, Träger-, Spreng- oder Schmiermittel oder Substanzen zur Erzielung einer Depotwirkung Anwendung finden.

Die folgenden Beispiele erläutern die Erfindung:

*Beispiel 1*

*2-(2-Chlor-4-cyclopropyl-phenyl-imino)-imidazolidin*

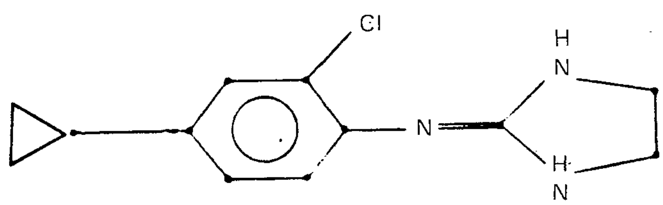

Mol-Gew.: 235,7
Summenformel: $C_{12}H_{14}ClN_3$
Schmp.: 135,5-138,5°C
Elementaranalyse:

| | | | | |
|---|---|---|---|---|
| Ber.: | C 61,15 | H 5,99 | Cl 15,04 | N 17,83 |
| Gef.: | C 61,07 | H 6,05 | Cl 15,42 | N 17,66 |

a) N-(2-Chlor-4-cyclopropyl-phenyl)-S-methyl-isothiouronium-jodid

4-Cyclopropylanilin wird zu 4-Cyclopropylacetanilid (Fp. 111-113.5°C) acetyliert und analog der Vorschrift in J. Amer. Chem. Soc. *62* (1940) 2103 zu 2-Chlor-4-cyclopropyl-anilin chloriert und verseift. Durch Umsetzung des Anilins mit Ammoniumthiocyanat und anschliessende Methylierung des erhaltenen N-(2-Chlor-4-cyclopropyl-phenyl)-thioharnstoffs mit Methyljodid erhält man die obige Verbindung.

b) 2-(2-Chlor-4-cyclopropyl-phenyl-imino)-imidazolidin

8,30 g N-(2-Chlor-4-cyclopropyl-phenyl)-S-methyl-isothiouronium-jodid werden in 25 ml Methanol zusammen mit 2,3 ml (150%) Äthylendiamin 15 Stunden lang am Rückfluss erhitzt. Hierauf wird die klare Reaktionsmischung im Vakuum eingedampft, wobei ein honigartiger Rückstand verbleibt. Diesen löst man in 1N HCl und extrahiert die salzsaure Lösung zweimal mit Äther. Die wässrige Phase wird anschliessend mit 2N NaOH auf pH 6 gepuffert und bei aufsteigenden pH-Werten (fraktioniertes Alkalisieren mit 2N NaOH) fraktioniert mit Äther extrahiert (50-ml-Portionen). Die annähernd einheitlichen Ätherfraktionen werden vereinigt (durch Dünnschichtchromatogramm-Kontrolle), über $MgSO_4$ getrocknet und im Vakuum eingedampft. Zur weiteren Reinigung wird das rohe 2-(2-Chlor-4-cyclopropyl-phenyl-imino)-imidazolidin über Kieselgel chromatographiert. Elutionsmittel: Isopropanol : Essigester : konz. Ammoniak = 50:50:1.

Ausbeute: 1,3 g (entsprechend 23,6% der Theorie).
Schmp.: 135,5-138,5°C
Dünnschichtchromatogramm:
System: Isopropanol : Essigester : konz. Ammoniak (50 : 50 : 1)

Träger: Kieselgel-Fertigplatten Merck-Darmstadt Nr. 60 F 254
Detektor: (a) UV, (b) Kaliumjodplatinat nach Schlittler
Rf: 0,3.

*Formulierungsbeispiele*

*Beispiel A: Dragées*

| | |
|---|---|
| Wirkstoff gemäss Erfindung | 5 mg |
| Milchzucker | 65 mg |
| Maisstärke | 130 mg |
| sek. Calciumphosphat | 40 mg |
| lösliche Stärke | 3 mg |
| Magnesiumstearat | 3 mg |
| kolloidale Kieselsäure | 4 mg |
| insgesamt | 250 mg |

Herstellung:

Der Wirkstoff wird mit einem Teil der Hilfsstoffe vermischt, intensiv mit einer wässrigen Lösung der löslichen Stärke durchgeknetet und in üblicher Weise mit Hilfe eines Siebes granuliert. Das Granulat wird mit dem Rest der Hilfsstoffe vermischt und zu Dragéekernen von 250 mg Gewicht verpresst, die dann in üblicher Weise mit Hilfe von Zucker, Talkum und Gummi arabicum dragiert werden.

*Beispiel B: Ampullen*

| | | |
|---|---|---|
| Wirkstoff gemäss Erfindung | | 1,0 mg |
| Natriumchlorid | | 18,0 mg |
| dest. Wasser | ad | 2,0 mg |

Herstellung:

Wirkstoff und Natriumchlorid werden in Wasser gelöst und unter Stickstoff in Glasampullen abgefüllt.

**Patentansprüche**

1. 2-(2-Chlor-4-cyclopropyl-phenyl-imino)-imidazolidin der Formel

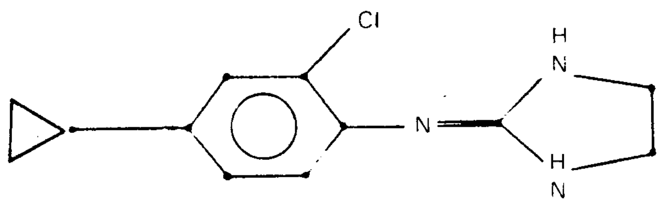

sowie dessen Säureadditionssalze.

2. Verfahren zur Herstellung von 2-(2-Chlor-4--cyclopropyl-phenyl-imino)-imidazolidin nach Anspruch 1 und von dessen Säureadditionssalzen, dadurch gekennzeichnet, dass man

a) eine Verbindung der allgemeinen Formel

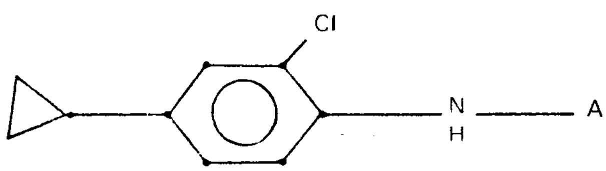

in der A eine Cyanogruppe oder den Rest 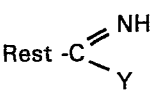 bedeutet, wobei Y eine Alkoxy- oder Alkylthiogruppe mit bis zu 4 C-Atomen oder eine Sulfhydryl- oder Aminogruppe darstellt, mit Äthylendiamin oder dessen Säureadditionssalzen umsetzt; oder

b) eine Verbindung der allgemeinen Formel

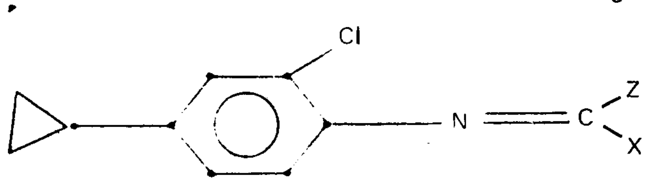

worin X und Z, die gleich oder verschieden sein können, ein Halogenatom oder eine Alkoxy- oder Alkylthiogruppe mit bis zu 4 C-Atomen bedeuten, mit Äthylendiamin umsetzt; oder

c) eine Verbindung der allgemeinen Formel

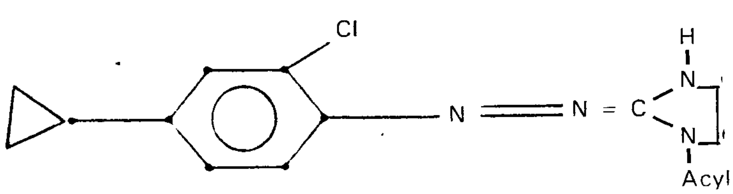

worin Acyl eine aliphatische oder aromatische Acylgruppe bedeutet, mit aliphatischen Alkoholen oder Mineralsäuren umsetzt und gegebenenfalls das erhaltene Endprodukt in ein Säureadditionssalz überführt.

3. Pharmazeutische Zubereitungen, dadurch gekennzeichnet, dass sie als Wirkstoff die Verbindung der Formel I nach Anspruch 1 oder deren Säureadditionssalze enthalten.

4. Verfahren zur Herstellung von Arzneimitteln nach Anspruch 3, dadurch gekennzeichnet, dass man die Verbindung der Formel I oder deren Säureadditionssalze mit üblichen galenischen Hilfs-, Träger-, Spreng- oder Schmiermitteln bzw. Substanzen zur Erzielung einer Depotwirkung zu Tabletten, Kapseln, Zäpfchen, Lösungen oder Pulver formuliert.

5. Verbindungen nach Anspruch 1 zur Anwendung bei der Behandlung von Coronarerkrankungen.

**Claims**

1. 2-(2-Chloro-4-cyclopropyl-phenyl-imino)-imid-azolidine of formula

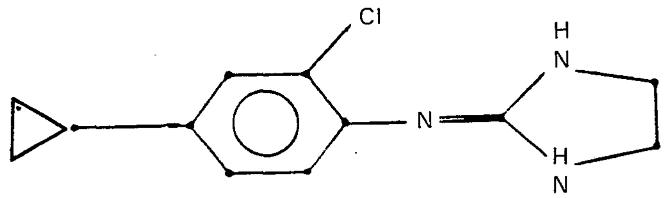

and the acid addition salts thereof.

2. Process for the preparation of 2-(2-chloro-4-cyclopropyl-phenyl-imino)-imidazolidine as claimed in claim 1 and of the acid addition salts thereof, characterised in that

a) a compound of general formula

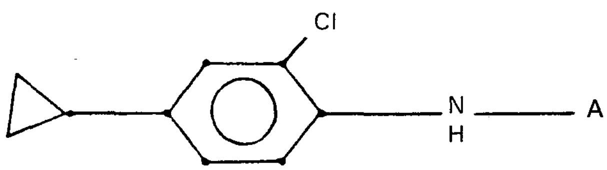

wherein A represents a cyano group or the group 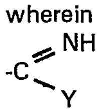 (wherein Y represents an alkoxy or alkylthio group with up to 4 carbon atoms or a mercapto or amino group) is reacted with ethylene diamine or the acid addition salts thereof; or

b) a compound of general formula

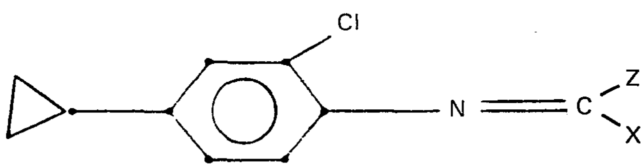

wherein X and Z, which may be the same or different, represent a halogen atom or an alkoxy or alkylthio group with up to 4 carbon atoms, is reacted with ethylene diamine; or

c) a compound of general formula

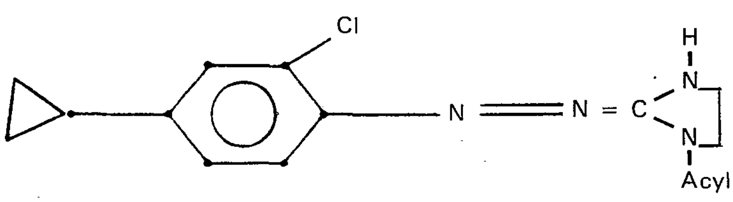

wherein Acyl represents an aliphatic or aromatic acyl group is reacted with aliphatic alcohols or inorganic acids and, if desired the end product obtained is converted into an acid addition salt.

3. Pharmaceutical preparations, characterised in that they contain, as active substance, the compound of formula I as claimed in claim 1 or the acid addition salts thereof.

4. Process for the preparation of pharmaceutical compositions as claimed in claim 3, characterised in that the compound of formula I or an acid addition salt thereof is combined with conventional galenic excipients, carriers, disintegrants or lubrificants or substances for obtaining delayed release to form tablets, capsules, suppositories, solutions or powders.

5. Compounds as claimed in claim 1, for use in the treatment of coronary diseases.

**Revendications**

1. 2-(2-chloro-4-cyclopropyl-phényl-imino-imidazolidine de formule

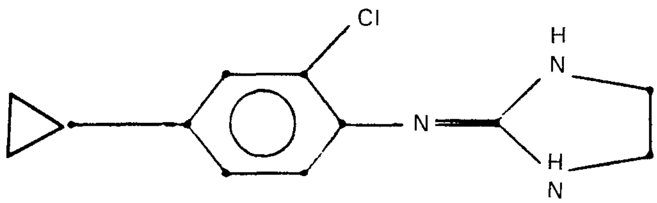

ainsi que ses sels d'addition d'acide.

2. Procédé pour la préparation de la 2-(2-chloro-4-cyclopropyl-phényl-imino)-imidazolidine selon la revendication 1 et de ses sels d'addition d'acide, caractérisé en ce que

a) on fait réagir un composé de formule générale

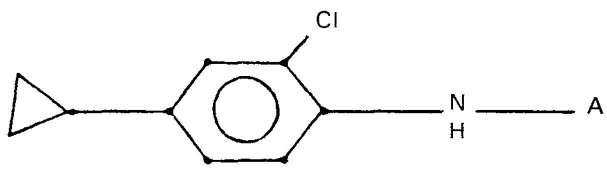

dans laquelle A représente un groupe cyano ou le radical 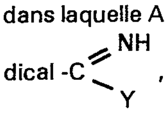 , Y représentant un groupe alcoxy ou alcoylthio avec jusqu'à 4 atomes de C ou un groupe sulfhydryle ou amino, avec l'éthylènediamine ou ses sels d'addition d'acide; ou

b) on fait réagir un composé de formule générale

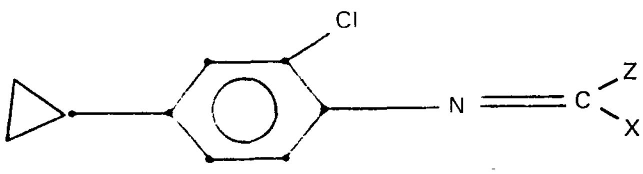

dans laquelle X et Z qui peuvent être identiques ou différents, représentent un atome d'halogène ou un groupe alcoxy ou alcoylthio avec jusqu'à 4 atomes de C, avec l'éthylènediamine; ou

c) on fait réagir un composé de formule générale

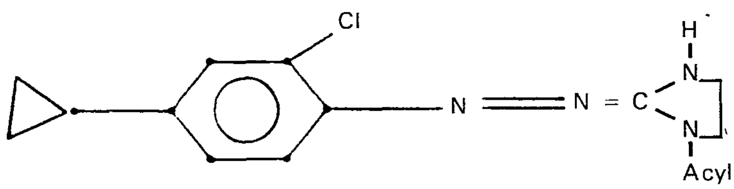

dans laquelle acyle représente un groupe acyle aliphatique ou aromatique, avec des alcools aliphatiques ou des acides minéraux et on transforme éventuellement le produit final obtenu en un sel d'addition d'acide.

3. Préparations pharmaceutiques, caractérisées en ce qu'elles contiennent en tant que substance active le composé de formule I selon la revendication 1 ou ses sels d'addition d'acide.

4. Procédé de préparation de médicaments selon la revendication 3, caractérisé en ce que l'on formule le composé de formule I ou ses sels d'addition d'acide avec des adjuvants, excipients, désagrégeants ou lubrifiants ou respectivement substances pour obtenir un effet retard galéniques, habituels en comprimés, capsules, suppositoires, solutions ou poudres.

5. Composés selon la revendication 1 pour l'utilisation dans le traitement des maladies des coronaires.